# EUROPÄISCHE PATENTANMELDUNG

(11) **EP 2 431 056 A1**
(43) Veröffentlichungstag der Anmeldung: **21.03.2012**
(21) Anmeldenummer: 10009703.9
(22) Anmeldetag: 16.09.2010
(51) Int. Cl.: A61L 2/03, A61K 33/14, C02F 1/461, A61K 9/00, A61L 12/10

(54) **Pharmazeutische Zusammensetzung, enthaltend elektrochemisch aktivierte Salzlösung**

(71) Anmelder: Caliopa AG, 6314 Unterägeri (CH)
(72) Erfinder: Mathé, Hans-Georg, 6330 Cham (CH)
(74) Vertreter: Tergau & Walkenhorst

(57) **Zusammenfassung**

Eine pharmazeutische Zusammensetzung, enthaltend einen Wirkstoff und einen Trägerstoff, bei der der Trägerstoff aus einer elektrochemisch aktivierten wässrigen Kochsalzlösung besteht und in einer vom Wirkstoff getrennten Phase vorliegt, soll ohne unzumutbare Beeinträchtigung der Wirksamkeit des Wirkstoffs eine besonders hohe Lagerfähigkeit und Langzeitstabilität der Zusammensetzung gewährleisten. Dazu weist erfindungsgemäß der Trägerstoff einen Gehalt an freiem Chlor von mehr als 500 mg / l, vorzugsweise von mehr als 600 mg / l, auf.

## Beschreibung

Die Erfindung bezieht sich auf pharmazeutische Zusammensetzungen, enthaltend einen Wirkstoff und einen Trägerstoff, wobei der Trägerstoff aus einer elektrochemisch aktivierten wässrigen Kochsalzlösung besteht.

Anolyt oder ASECA-Lösung ist eine mit dem in der nicht vorveröffentlichten EP 10 003 555.9 beschriebenen ASECA-Verfahren hergestellte neutrale, elektrochemisch aktivierte Salz-Lösung, die ein hochwirksames Desinfektionsmittel ist. Diese aktivierte Lösung kann durch Elektrolyse von Natriumchlorid-Lösungen gewonnen werden. Es kann in Anwendungen zur Flächendesinfektion eingesetzt werden, z.B. von Arbeitsplatten, Tischen, Böden, etc., zur Kaltdesinfizierungshandlungen, in der Landwirtschaft für die Beseitigung von mikrobiellen Organismen, zum Wäsche Waschen und in Anwendungen in Schwimmbädern und sogar als Prophylaxe gegen Fußpilz. Ein Gerät zur Herstellung von neutralen elektrochemisch aktivierten Salzlösungen im allgemeinen ist beispielsweise in der EP-A-1 728 768 beschrieben.

Aus der EP 2 178 501 ist eine pharmazeutische Zusammensetzung der oben genannten Art bekannt, die zusätzlich zu einem Wirkstoff einen vom Wirkstoff in einer separaten, getrennten Phase vorgehaltenen Trägerstoff umfasst. Der Trägerstoff besteht dabei aus einer elektrochemisch aktivierten wässrigen Kochsalzlösung und weist im Hinblick auf eine möglichst weitgehende Kompatibilität und Koexistenz mit der den Wirkstoff enthaltenden zweiten Phase einen Gehalt an freiem Chlor zwischen 1 und 500 mg/l und ein Redoxpotential zwischen 150 und 1350 mV auf.

Der vorliegenden Erfindung liegt nunmehr die Aufgabe zugrunde, eine pharmazeutische Zusammensetzung der genannten Art derart weiterzubilden, dass ohne unzumutbare Beeinträchtigung der Wirksamkeit des Wirkstoffs eine besonders hohe Lagerfähigkeit und Langzeitstabilität der Zusammensetzung gewährleistet ist.

Diese Aufgabe wird erfindungsgemäß dadurch gelöst, dass der Trägerstoff einen Gehalt an freiem Chlor von mehr als 500 mg/l, vorzugsweise von mehr als 600 mg/l, aufweist.

Die Erfindung geht von der Überlegung aus, dass gerade unter Rückgriff auf die aus der EP 2 178 501 grundsätzlich bekannte Vorhaltung des Wirkstoffs in einer vom Trägerstoff getrennten Phase in der Art einer Einbettung des Wirkstoffs in ihn umgebendes Trägermaterial eine gezielte Steigerung der Lagerbeständigkeit erreichbar ist, indem der Trägerstoff zusätzlich zu seinen Trägereigenschaften auf eine besonders weitgehende Konservierungswirkung ausgelegt wird. Dies ist erreichbar, indem der Trägerstoff als Biozid oder dergleichen ausgestaltet wird, wobei gerade aufgrund der Einbettung des Wirkstoffs in den umgebenden Trägerstoff in der Art einer Matrix eine zuverlässige Abschottung des Wirkstoffs gegenüber Keimen oder dergleichen sichergestellt werden kann.

Dazu sollte der Trägerstoff unter Abkehr von den in der EP 2 178 501 vorgesehenen Parametern gezielt und in erster Linie auf eine besonders hohe Biozidität oder Keimtötungswirkung ausgelegt sein. Um dies zu gewährleisten, sollte ein Gehalt an freiem Chlor im Trägerstoff von mehr als 500 mg/l, vorzugsweise von mehr als 600 mg/l, eingestellt werden. Besonders günstig ist dabei ein Gehalt an freiem Chlor von 700 mg/l oder mehr, da gerade ab dieser Grenze der Trägerstoff zur Erfüllung der Erfordernisse für Sterilität geeignet ist und somit eine besonders weitgehende Konservierungswirkung aufweist. Gerade mit einer derartigen Ausgestaltung ist somit die gewünschte Keimunterdrückung besonders zuverlässig erreichbar, und insbesondere das gängige Erfordernis an ein Desinfektionsmittel, nämlich die Reduktion eines anfänglich vorgegebenen Keimgehalts mindestens um den Faktor 100 000, ist ohne Weiteres ermöglicht.

Der genannte Gehalt an freiem Chlor kann dabei insbesondere durch Titration in an sich gängiger Weise ermittelt werden.

Eine zur Verwendung als Trägerstoff in der genannten pharmazeutischen Zusammensetzung geeignete elektrochemisch aktivierte wässrige Kochsalzlösung kann beispielsweise besonders vorteilhaft hergestellt werden durch Elektrolyse mit einer kaskadenartigen Verschaltung von Anodenräumen mehrerer Elektrolysezellen, wie dies beispielsweise beschrieben ist in der nicht vorveröffentlichten EP 10 003 555.9. Die Offenbarung dieser Druckschrift insbesondere hinsichtlich der Herstellung einer elektrochemisch aktivierten wässrigen Kochsalzlösung mit besonders hohem Gehalt an freiem Chlor wird ausdrücklich mit einbezogen ("incorporation by reference"). Gerade bei dem dort beschriebenen Herstellungsverfahren steht als weiterer Herstellungsparameter neben den für den eigentlichen Elektrolyseprozess charakterisitischen Parametern (insbesondere Elektrolysestrom und -spannung) auch der Salzgehalt in der als Elektrolysemedium verwendeten Kochsalzlösung zur Verfügung. Dieser Salzgehalt unterliegt zwar einigen Randbedingungen (insbesondere sollte er ausreichend hoch bemessen sein, um bei gängiger Stromstärke des Elektrolysestroms ausreichend hohe Produktionsraten an Chlor im Anolyten zu gewährleisten), kann abgesehen davon aber im wesentlichen in der Art eines freien Parameters geeignet gewählt werden. Insbesondere kann der Salzgehalt geeignet eingestellt werden, um im Endprodukt eine gewünschte Osmolarität einzustellen und damit ein in dieser Hinsicht an den vorgesehenen Einsatzzweck angepasstes Produkt (z. B. isotonisch) bereitzustellen.

Das Redoxpotential der als Trägerstoff vorgesehenen elektrochemisch aktivierten Salzlösung, insbesondere ermittelbar durch amperometrische Messungen, beträgt bei pH-neutralen Lösungen vorteilhafterweise mindestens zwischen +600 mV und + 1000 mV, vorzugsweise mindestens + 800 mV und/oder vorzugsweise bis + 900 mV, kann bei sauren Lösungen (also niedrigem pH-Wert von beispielsweise etwa 2,5) aber auch deutlich höhere Werte annehmen. Damit ist unter anderem eine besonders günstige Langzeitstabilität des Stoffs gegeben. Die elektrochemisch aktivierte Salzlösung ist vorzugsweise eine Alkalimetall-Hypochlorit-Lösung, z. B. eine Lithium-, Natrium- oder Kalium-Hypochloritlösung. Besonders bevorzugt ist die Lösung eine Natriumhypochlorit-Lösung.

Die elektrochemisch aktivierte Kochsalzlösung hat vorteilhafterweise einen pH-Wert von 2 bis 8, besonders bevorzugt von etwa 7. In bestimmten Ausführungsformen kann auch ein pH-Wert von 2 bis 5, z. B. 2 bis 4 , 2 bis 3 oder 2 bis 2,8, günstig sein. Eine derartige, saure Darstellung des Trägerstoffs gerade in Kombination mit dem genannten Gehalt an freiem Chlor ist besonders vorteilhaft bei einem Einsatz des Trägerstoffs als Biozid gegen Bakteriensporen. In anderen günstigen Ausführungsformen kann der pH-Wert auch 5 bis 8, insbesondere 5,9 bis 7,6 und insbesondere von 6,7 bis 7,4, gewählt sein.

Der Inhalt von Chlorat und/oder von Chlorit liegt vorzugsweise unter toxischen Mengen, insbesondere bei weniger als 10 mg/l Chlorit bzw. weniger als 20 mg/l Chlorat. Darüber hinaus ist die Lösung vorzugsweise frei von nachweisbaren Mengen von Radikalen wie OH-Radikalen oder Ozon. Darüber hinaus ist die Lösung bevorzugt frei von Schwermetall-lonen wie beispielsweise von Mo-lonen.

Als Wirkstoff kann grundsätzlich jedes für den Einsatz in der Human- oder Veterinärmedizin geeignete Medikament vorgesehen sein. Insbesondere kann ein hydrophiler oder ein lipophiler Wirkstoff und/oder eine Kombination derartiger Wirkstoffe vorgesehen sein. In bestimmten Ausführungsformen ist es bevorzugt, dass der Wirkstoff aus lipophilen oder amphiphilen Zutaten, also Zutaten, die einen Butanol-Wasser-Verteilungskoeffizienten von mindestens 0,5, vorzugsweise von mindestens 1, haben, ausgewählt ist. In anderen bevorzugten Ausführungsformen umfasst der Wirkstoff einen hydrophilen Inhaltsstoff wie Polysaccharid. Zum Beispiel kann der Wirkstoff ausgewählt sein aus Wirkstoffen zur Behandlung von Glaukom, z. B. Prostaglandine wie Latanoprost, Beta-Blocker wie Timolol, Mittel zur Senkung von erhöhtem Augeninnendruck wie Dorzolamid, Wirkstoffen zur Behandlung des Syndroms des trockenen Auges (ophthalmologische Befeuchtungsmittel), wie Hydroxypropylmethylcellulose (Hypromellose), Hyaluronsäure und anderen pharmazeutischen Wirkstoffen.

Die pharmazeutische Zusammensetzung der vorliegenden Erfindung ist gegen mikrobiellen Abbau stabilisiert. So ist die Zusammensetzung auch geeignet für Multi-Use-Anwendungen. Aufgrund der Eigenschaften des Trägerstoffs, insbesondere dessen hohe Biozidität, kann die Zusammensetzung für multi-use-Anwendungen sogar ganz ohne konventionelle Konservierungsstoffe ausgeführt sein.

Das Präparat hat eine Stabilität gegen mikrobiellen Abbau von vorzugsweise mindestens 6 Monaten, mehr bevorzugt von mindestens 12, insbesondere 24 oder mehr, Monaten, selbst bei Lagerung bei Raumtemperatur. Die anti-mikrobielle Aktivität der elektrochemisch aktivierten Salzlösung kann beispielsweise durch Messung des Produkts c x t aus der Konzentration (c) und der Einwirkungszeit (t) nach einer Methode bestimmt werden, wie beschrieben von Schleupen, GWF, 1996. Vorzugsweise wird ein Wert für c x t von 1 mg/l x min oder weniger, besonders bevorzugt von 0,5 mg/l x min oder weniger, eingestellt, um Reduktionsraten von 10⁶ gegen Mikroorganismen wie Pseudomonas aeruginosa oder Legionella pneumophila zu erhalten.

Das Präparat kann für jede Art von Anwendung, z.B. für lokale oder systemische Verabreichung, vorgesehen sein. Zum Beispiel ist die Zusammensetzung für die Augen-, Nasen-, Ohr-, topische, pulmonale, Schleimhaut-, orale oder intraperitoneale Applikation, z. B. für die Verabreichung durch Injektion, geeignet. Bevorzugte Zusammensetzungen sind für okulare Verabreichung, z. B. für die Behandlung von Glaukom oder des Syndroms des trockenen Auges, vorgesehen.

Die Zusammensetzung bildet vorzugsweise mindestens zwei getrennte Phasen, wobei der Wirkstoff in einer ersten Phase und die elektrochemisch aktivierte Salzlösung in einer zweiten Phase getrennt von der ersten Phase vorliegt. Die erste Phase kann eine feste Partikel-Phase, eine flüssige hydrophobe Phase oder eine feste oder flüssige Phase mit einer Barriere gegenüber der zweiten Phase sein, wobei die zweite,durch den Trägerstoff gebildete Phase vorzugsweise eine wässrige Phase ist. So kann die Zusammensetzung eine Emulsion , z. B. eine Mikroemulsion oder eine liposomale Zusammensetzung oder eine Mikrokapsel-Zusammensetzung oder Dispersion sein. Der Wirkstoff kann dabei emulgiert oder dispergiert in Gegenwart des Trägers, z. B. eines lipophilen Trägers und/oder eines Tensids im wässrigen Träger, vorliegen. Der Wirkstoff kann dabei physisch von der elektrochemisch aktivierten Salzlösung getrennt vorliegen.

Besonders bevorzugt sind Mikroemulsionen wie beschrieben in EP 07 008 347,1, die hierin durch Bezugnahme aufgenommen ist ("incorporation by reference").

Neben dem Wirkstoff und der als Trägerstoff vorgesehenen elektrochemisch aktivierten Salzlösung kann die Zusammensetzung auch noch weitere, an sich gängige Zusätze enthalten, wie z.B. Puffer, Hilfsstoffe, Füllstoffe, Verdünnungsmittel, etc.

Die erfindungsgemäße pharmazeutische Zusammensetzung kann bevorzugt verwendet werden in der Human- oder Tiermedizin.

Noch ein weiterer Aspekt der vorliegenden Erfindung bezieht sich auf die Verwendung einer elektrochemisch aktivierten Salzlösung der oben genannten Art für die Reinigung von Kontaktlinsen, z. B. Glas- oder Kunststoff Kontaktlinsen. In dieser Ausführungsform kann die Lösung ohne jeden Wirkstoff vorliegen oder einen Wirkstoff enthalten, z. B. ein Polymer, wie oben beschrieben.

Ausführungsbeispiele der Erfindung werden nachfolgend näher beschrieben.

Es wurden verschiedene Varianten einer elektrochemisch aktivierten Kochsalzlösung nach dem in der EP 10 003 555.9 beschriebenen Verfahren, insbesondere mit kaskadenartigem Durchlauf des Anolyten durch zwei oder mehr Anodenräume der Elektrolyseanlage, hergestellt. Durch Variation der Elektrolyseparameter (Elektrolysestrom und -spannung) wurden verschiedene Werte an freiem Chlor zwischen 1200 mg/l und 1800 mg/l eingestellt. In der so hergestellten Lösung wurde ein pH-Wert von etwa 7 eingestellt. Zudem wurde eine saure Variante des Anolyten mit einem pH-Wert von etwa 2,5 hergestellt.

Die biozide oder keimtötende Wirkung und somit die antimikrobielle Aktivität des Anolyten wurde gegen Beispiele aus sämtlichen bekannten Keimgruppen, nämlich gegen Bakterien, Bakteriensporen, unbehüllte Viren, behüllte Viren sowie Pilze und Pilzsporen, getestet. Insbesondere wurden Versuche mit Staphylococcus aureus, Enterococcus hirae, Eschericia coli, Proteus mirabilis, Pseudomonas aeruginosa, Canida albicans, Aspergillus niger, Mycobacterium terrae und Mycobacterium avium durchgeführt. Dabei wurde nach einer Kultivierung über 48 h bei einer Temperatur von 37°C/30°C einerseits ermittelt, bei welcher Konzentration des Anolyten in wässriger Lösung nach einer Inkubationszeit von 1 min bzw. 5 min ein Wachstum und eine organische Aktivität nicht mehr nachweisbar ist. Andererseits wurde ermittelt, nach welcher Zeit in 100% Lösung des Anolyten das mikrobieller Wachstum nach einer Inkubation auf keramischer Oberfläche bei einer organischen Anfangsbelastung von 0,3 o/oo vollständig zum Erliegen kommt.

Dabei wurden folgende Ergebnisse festgestellt:

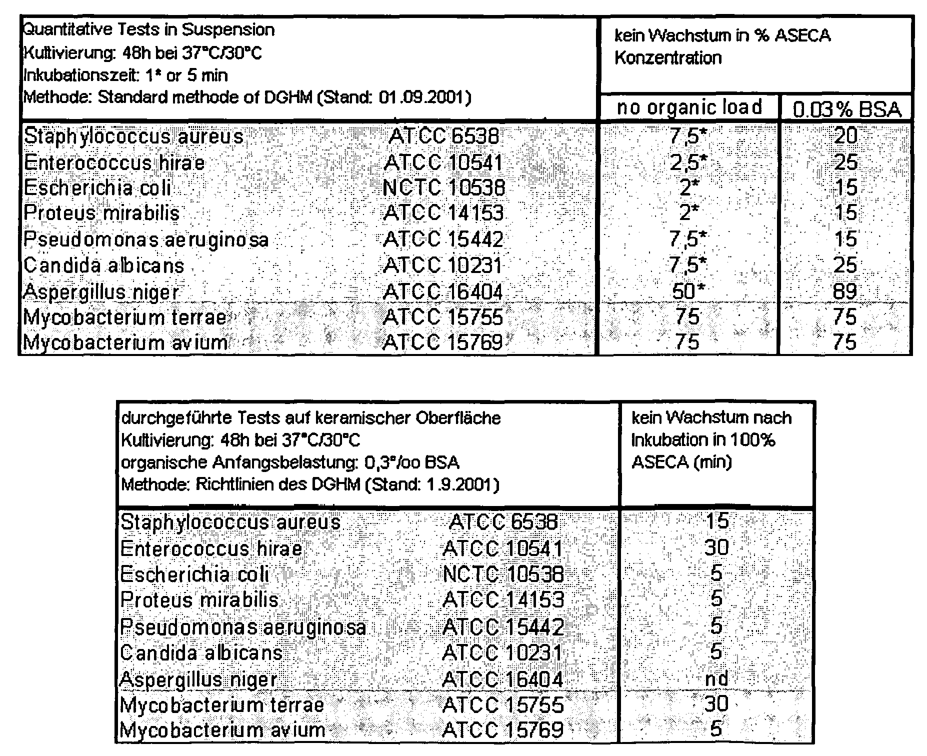

Nach einer Behandlungsdauer von maximal 30 Minuten war somit in jedem Fall keinerlei organisches Wachstum mehr beobachtbar. Die als Trägerstoff vorgesehene elektrochemisch aktivierte Salzlösung weist somit eine vergleichweise hohe und umfassende, also breitbandige und gegen sämtliche Keimgruppen feststellbare, biozide Wirkung auf.

Auch gegen virale Belastung ist eine derartige hohe Wirksamkeit feststellbar. Die folgende Tabelle gibt die Zeitspanne an, nach der eine Reduktion der Virenzahl um den Faktor 10 000 (log4-Reduktion) in einer 80%-Lösung des Anolyten vorlag:

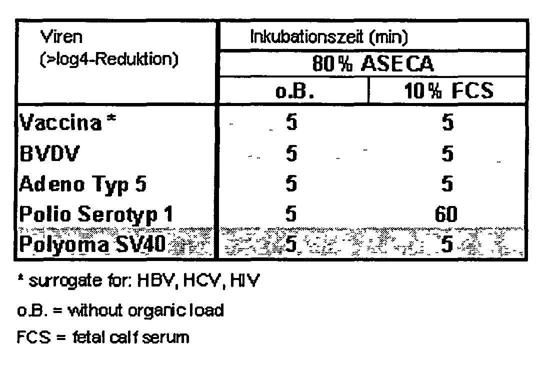

## Patentansprüche

1. Pharmazeutische Zusammensetzung, enthaltend einen Wirkstoff und einen Trägerstoff, wobei der Trägerstoff aus einer elektrochemisch aktivierten wässrigen Kochsalzlösung besteht und in einer vom Wirkstoff getrennten Phase vorliegt, und wobei der Trägerstoff einen Gehalt an freiem Chlor von mehr als 500 mg / l, vorzugsweise von mehr als 600 mg / l, aufweist.

2. Pharmazeutische Zusammensetzung nach Anspruch 1, deren Trägerstoff ein Redoxpotential zwischen 600 und 1000mV, vorzugsweise zwischen 800 und 900 mV, aufweist.

3. Pharmazeutische Zusammensetzung nach Anspruch 1 oder 2, deren Trägerstoff durch eine Natriumhypochlorit-Lösung gebildet ist.

4. Pharmazeutische Zusammensetzung nach einem der Ansprüche 1 bis 3, deren Trägerstoff einen pH-Wert von 2 bis 8, vorzugsweise von etwa 7, aufweist.

5. Pharmazeutische Zusammensetzung nach einem der Ansprüche 1 bis 4, deren Trägerstoff einen Gehalt an Chlorat und/oder Chlorit von weniger als 10 mg / l aufweist.

6. Pharmazeutische Zusammensetzung nach einem der Ansprüche 1 bis 5, wobei der Wirkstoff ausgewählt ist aus der Gruppe der Prostaglandine, Beta-Blocker, Mittel zur Senkung erhöhten Augeninnendruck oder ophthalmologische Befeuchtungsmittel.

7. Pharmazeutische Zusammensetzung nach einem der Ansprüche 1 bis 6, die für Multi-Use-Anwendungen vorgesehen ist.

8. Pharmazeutische Zusammensetzung nach einem der Ansprüche 1 bis 7, die für eine lokale oder systemische Verabreichung vorgesehen ist.

9. Pharmazeutische Zusammensetzung nach einem der Ansprüche 1 bis 8, die für Augen-, Nasen-, Ohr-, topische, pulmonale, Schleimhaut-, orale oder intraperitoneale Verabreichung vorgesehen ist.

10. Pharmazeutische Zusammensetzung nach einem der Ansprüche 1 bis 9, in der der Wirkstoff in Form einer Phase fester Partikel, einer flüssigen hydrophoben Phase oder einer festen oder flüssigen Phase mit einer Barriere gegenüber der elektrochemisch aktivierten Kochsalzlösung vorhanden ist.

11. Pharmazeutische Zusammensetzung nach einem der Ansprüche 1 bis 10, die eine Emulsion oder Dispersion ist.

12. Verwendung einer wässrigen elektrochemisch aktivierten Salzlösung mit einem Gehalt an freiem Chlor von mehr als 500 mg / l als Träger für die Herstellung einer pharmazeutischen Zubereitung.

13. Pharmazeutische Zusammensetzung, enthaltend ein ophthalmisches Befeuchtungsmittel und einen Trägerstoff, wobei der Trägerstoff aus einer elektrochemisch aktivierten wässrigen Kochsalzlösung besteht und einen Gehalt an freiem Chlor von mehr als 500 mg / l, vorzugsweise von mehr als 600 mg / l, aufweist.

14. Pharmazeutische Zusammensetzung nach Anspruch 13, die eine wässrige Lösung ist.

15. Pharmazeutische Zusammensetzung nach Anspruch 13 oder 14 , wobei das ophthalmische Befeuchtungsmittel ein Polysaccharid oder ein Polyvinylpyrrolidon-Polymer wie beispielsweise Cellulose, ein Cellulosederivat oder ein Glucosamin-Glycan, wie beispielsweise Hyaluronsäure, ist.

16. Pharmazeutische Zusammensetzung nach einem der Ansprüche 13 bis 15, wobei keine weiteren Wirkstoffe und/oder Konservierungsstoffe vorhanden sind.

17. Verwendung der pharmazeutischen Zusammensetzung nach einem der Ansprüche 13 bis 16 zur Behandlung und/oder zur Verhütung trockener Augen.
